# EUROPEAN PATENT APPLICATION

(11) **EP 4 625 434 A1**
(43) Date of publication of application: **01.10.2025**
(21) Application number: 24305484.8
(22) Date of filing: 28.03.2024
(51) Int. Cl.: G16H 40/63, G16H 30/20, G16H 30/40, A61B 34/20, A61B 34/00

(54) **SUPPORTING AN INTERVENTIONAL PROCEDURE**

(71) Applicant: Koninklijke Philips N.V., 5656 AG Eindhoven (NL)
(72) Inventor: SCHAEFER, Dirk, 5656 Eindhoven (NL); WISSEL, Tobias, 5656 Eindhoven (NL); GRASS, Michael, 5656 Eindhoven (NL); HENDRIKS, Bernardus, 5656 Eindhoven (NL); FLORENT, Raoul, 5656 Eindhoven (NL); OLIVAN BESCOS, Javier, 5656 Eindhoven (NL); LUCASSEN, Gerald, 5656 Eindhoven (NL); GROEN, Joanneke, 5656 Eindhoven (NL); LARUE, Ruben, 5656 Eindhoven (NL); LAI, Marco, 5656 Eindhoven (NL)
(74) Representative: Philips Intellectual Property & Standards

(57) **Abstract**

A system for supporting an interventional procedure to be performed at a treatment site within a subject, is provided. The system comprises one or more processors configured to: retrieve, from a database, subject data (130_{1..i}) for the subject of the interventional procedure. The subject data relates to one or more positions (140_{1..j}) along an access path (150) to the treatment site (160) within the subject. The one or more processors are also configured to output the subject data (130_{1..i}).

## Description

### TECHNICAL FIELD

The present disclosure relates to supporting an interventional procedure to be performed at a treatment site within a subject. A system, a computer-implemented method, and a computer program product, are disclosed.

### BACKGROUND

Over the course of a subject's life, a variety of medical imaging procedures may be performed on a subject. For instance, computed tomography "CT", X-ray projection, magnetic resonance imaging "MRI", and ultrasound, imaging procedures may be performed on a subject. Similarly, other types of medical data may be acquired for the subject. For instance, intravascular measurements of calcium burden, blood flow, and so forth, may be acquired for the subject. Such medical data, referred to herein as "subject data", may be stored in various databases. If an interventional procedure is to be performed on the subject, this previously-acquired subject data can be a valuable source of information that can be used to support the procedure.

By way of an example, the treatment of patients with coronary artery disease "CAD" typically involves the acquisition of a diagnostic Coronary Computed Tomography Angiography "CCTA" image. The CCTA image is used in both the diagnosis, and also the planning, of a percutaneous coronary intervention "PCI". The CCTA image is often used to create a three-dimensional reconstruction of the coronary vessels, for instance to characterize plaque in the vessels, to obtain quantitative blood flow measurements such as the virtual fractional flow reserve "FFRCT", and to determine locations suitable for stent placement. Other subject data may also have been acquired for the subject. For instance, if a calcified stenosis in the coronary artery has been treated during a historic interventional procedure, information about the cracking of calcium deposits, the type of balloon and stent that have been used to treat the calcium deposits, how the vessel was approached during treatment, and so forth, will also have been acquired. This information can be important for future procedures.

However, there are challenges to using such subject data. The subject data is often acquired using different types of medical imaging systems, different types of medical devices, and so forth. Individual sources of subject data may only cover specific parts of a subject's anatomy. Consequently, physicians face a challenge in assembling the subject data such that it can be used to support a new interventional procedure. As a result, previously-acquired subject data is often not used to its fullest extent.

Thus, there remains a need for improvements in the way in which previously-acquired subject data is used to support interventional procedures.

### SUMMARY

According to one aspect of the present disclosure, a system for supporting an interventional procedure to be performed at a treatment site within a subject, is provided. The system includes one or more processors configured to:
retrieve, from a database, subject data for the subject of the interventional procedure, the subject data relating to one or more positions along an access path to the treatment site within the subject; and
output the subject data.

Since the system retrieves the subject data for the one or more positions along an access path to the treatment site within the subject, the system provides a physician with relevant information relating to the interventional procedure in an intuitive manner. By supporting the physician in performing the interventional procedure, efficient use is made of the subject data. This may in-turn lead to benefits such as a reduction in the number of imaging procedures, a reduction in the amount of contrast agent usage, and so forth.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for supporting an interventional procedure to be performed at a treatment site within a subject, in accordance with some aspects of the present disclosure.
Fig. 2 is a flowchart illustrating an example of a computer-implemented method of supporting an interventional procedure to be performed at a treatment site within a subject, in accordance with some aspects of the present disclosure.
Fig. 3 is an example of a graphical representation 210 of a subject and a graphical representation of subject data 130_{1..7} relating to one or more positions 140_{1..7} along an access path 150 to a treatment site 160 within the subject, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, in a corresponding manner.

In the following description, reference is made to examples of a system for supporting an interventional procedure to be performed at a treatment site within a subject. Reference is made to examples in which the interventional procedure is a cardiovascular procedure. It is, however, to be appreciated that the cardiovascular procedure serves only as an example, and that the system may alternatively be used to support other types of interventional procedures. In some examples, the system is used to support endoluminal procedures. In these examples, the access path may be referred to as an endoluminal access path. In this regard, the system may be used to support endovascular procedures, such as the example cardiovascular procedure, peripheral endovascular procedures, neurovascular procedures, and also other procedures that are performed within the vasculature and wherein the access path is an endovascular access path. The system may also be used to support other types of endoluminal procedures, such as endoscopic procedures, such as bronchoscopy procedures, colonoscopy procedures, laparoscopy procedures, and so forth. The system may alternatively be used to support other types of interventional procedures in which the access path is not necessarily an endoluminal access path. For instance, in other examples the system may be used to support needle interventional procedures, such as percutaneous biopsy procedures.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact diskread only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

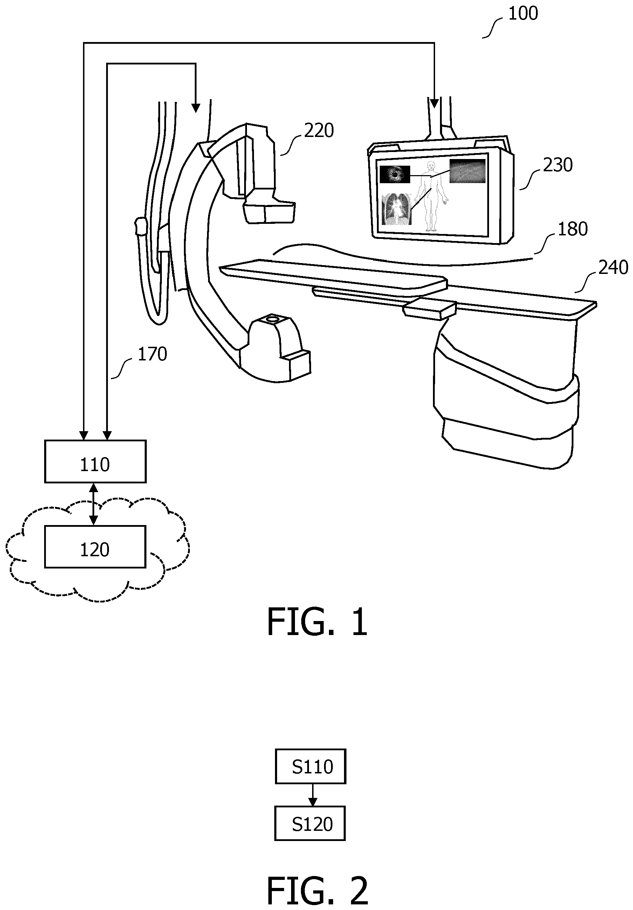

As mentioned above, there remains a need for improvements in the way in which previously-acquired subject data is used to support interventional procedures. Fig. 1 is a schematic diagram illustrating an example of a system 100 for supporting an interventional procedure to be performed at a treatment site within a subject, in accordance with some aspects of the present disclosure. Fig. 2 is a flowchart illustrating an example of a computer-implemented method of supporting an interventional procedure to be performed at a treatment site within a subject, in accordance with some aspects of the present disclosure. It is noted that operations that are described as being performed by the one or more processors of the system 100 illustrated in Fig. 1, may also be performed in the method illustrated in Fig. 2. Likewise, operations that are described in relation to the method described with reference to Fig. 2 may also be performed by the one or more processors of the system 100 illustrated in Fig. 1. With reference to Fig. 1, and Fig. 2, the system 100 for supporting an interventional procedure to be performed at a treatment site within a subject, comprises one or more processors 120 configured to:
retrieve S110, from a database 120, subject data 130_{1..i} for the subject of the interventional procedure, the subject data relating to one or more positions 140_{1..j} along an access path 150 to the treatment site 160 within the subject; and
output S120 the subject data 130_{1..i}.

Since the system retrieves the subject data for the one or more positions along an access path to the treatment site within the subject, the system provides a physician with relevant information relating to the interventional procedure in an intuitive manner. By supporting the physician in performing the interventional procedure, efficient use is made of the subject data. This may in-turn lead to benefits such as a reduction in the number of imaging procedures, a reduction in the amount of contrast agent usage, and so forth.

The system 100 may be used to support various types of interventional procedures. For instance, the system may be used to support a cardiovascular procedure, as described in the examples below. More generally, the system 100 may be used to support endoluminal procedures. For instance, the system 100 may be used to support an endovascular procedure, or an endoscopic procedure.
The operations that are performed by the one or more processors 110 of the system 100 are described in more detail below.

In the operation S110, subject data 130_{1..i} for the subject of the interventional procedure, is retrieved from a database 120.

In general, the subject data 130_{1..i} that is received in the operation 5110 includes medical data for the subject. The medical data may include one or more of the following: medical image data, measurement data, simulation data, risk data, data identifying a recommended treatment device, and outcome data representing an outcome of one or more historical interventional procedures performed on the subject. Examples of medical image data include Computed Tomography "CT" image data, X-ray projection image data, Magnetic Resonance Imaging "MRI" data, positron emission tomography "PET" image data, Single Photon Emission Computed Tomography "SPECT" image data, ultrasound image data, Intravascular ultrasound "IVUS" image data, Optical Coherence Tomography "OCT" image data, (hyperspectral) endoscopic image data, and so forth. In the example of endoscopy, examples of the medical image data further include endoscopic camera image data. Examples of measurement data include a geometric measurement of a lumen, such as a lumen diameter, a vascular stenosis length, a measurement of blood flow within a vessel, a measurement of intravascular calcium burden within a vessel, e.g. a calcium score, and so forth. Examples of simulation data include a simulated blood flow measurement for a blood vessel, such as a simulated Fractional Flow Reserve "FFR" measurement, simulation data relating to aneurysm rupture (e.g. as obtained from a biomechanical model). Examples of risk data include a risk of cardiovascular disease.

In general, the database 120 from which the subject data is received in the operation 5110 may be provided by a computer readable storage medium. The database 120 may be located locally, or remotely, with respect to the one or more processors 110. The database 120 may be distributed in the sense that it may be distributed across multiple computer readable storage media. In one example, the database comprises a Picture Archiving and Communication Systems "PACS" database. In one example, the database 120 is provided by the Cloud, as illustrated in Fig. 1.

The subject data 130_{i..j} that is received in the operation S110, relates to one or more positions 140_{1..j} along an access path 150 to a treatment site 160 within a subject.

The treatment site may be in various anatomical locations. In some examples, the treatment site is in an endoluminal location. For instance, the treatment site may be an endovascular treatment site, such as a cardiovascular treatment site, or a neurovascular treatment site. By way of some further examples, the treatment site may alternatively be located in another type of lumen, such as in the respiratory tract, or in the gastrointestinal tract.

In general, the access path extends between an entry point in the anatomy, to the treatment site. In some examples, at least a portion of the access path is defined by one or more lumens within the anatomy. In these examples, the access path may be referred-to as an endoluminal access path. For instance, in the examples mentioned above, at least a portion of the access path may extend along a portion of the vasculature, or the respiratory tract, or the gastrointestinal tract. Various corresponding entry points may be used, depending on the type of the treatment site. For instance, in the case of endovascular treatment sites, the corresponding entry point may be in the groin area, or in the wrist area, or in the clavicular area, for example. In the case of treatment sites that are located in the respiratory tract, or in the gastrointestinal tract, the entry point may be the mouth, or the anus, for example.

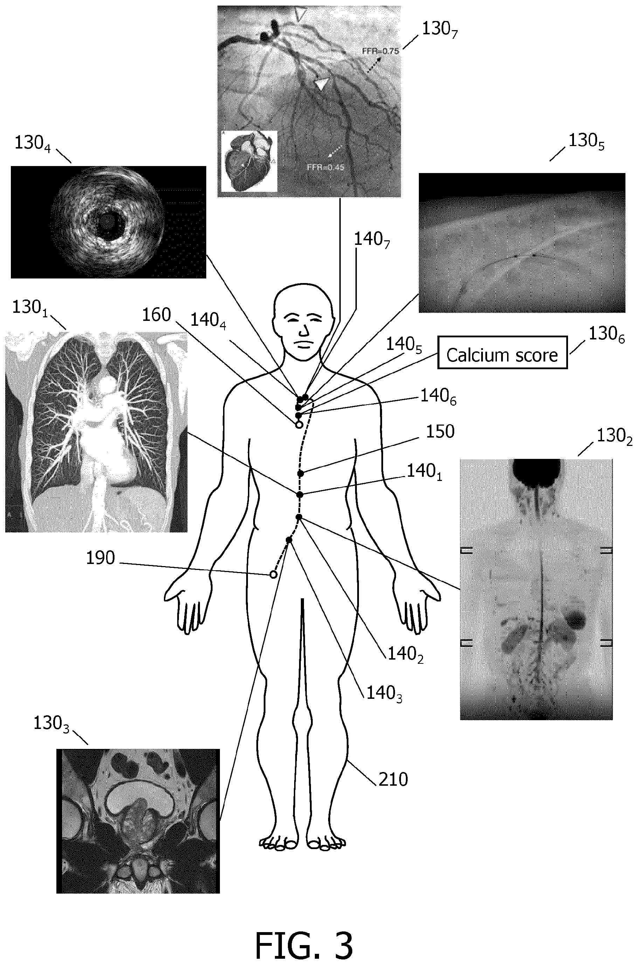

In some examples, the subject data is retrieved, and also stored, using a subject atlas. A subject atlas is a representation of a subject's anatomy. A subject atlas may be a textual, or a graphical representation of the subject's anatomy. In the latter case, a subject atlas may be a 2D, or a 3D, representation of a subject. An example of a subject atlas is the graphical representation 210 illustrated in Fig. 3.

In one example, the subject data 130_{1..i} comprises an indication of an origin of the subject data with respect to a subject atlas. In this example, the operation of retrieving S110 the subject data 130_{1..i}, is performed based on the indication of the origin of the subject data.

In this example, the origin of the subject data with respect to the subject atlas may be provided in the form of a label. For instance, an anatomical label such as "left anterior descending artery (LAD) artery", "left circumflex (LCX) artery", and so forth, may applied to the subject data. The database may store the subject data hierarchically based on the anatomical label such that together the anatomical labels provide the subject atlas. For instance, the body may be sub-divided into anatomical regions such as the head, arms, legs, torso, and abdomen, and within each anatomical region, further subdivisions may be provided such as bones, muscle, vasculature, and so forth. Together these anatomical regions represent the subject's anatomy, or in other words, a subject atlas. In this case, the anatomical labels that are applied to the subject data may then be used to retrieve the subject data from the database 120. A search of the database may be performed using the anatomical label in order to retrieve the subject data. For example, if a neurovascular intervention with femoral access is planned, then subject data along the access path to the treatment site relating to the femoral arteries, the aorta, and the carotid arteries, may be retrieved. If a cardiovascular intervention for (structural) heart disease is planned, then subject data along the access path to the treatment site relating to various vessels, cardiac chamber(s), cardiac valve(s), may be retrieved.

The indication of the origin of the subject data with respect to the subject atlas may alternatively be provided in a different manner. For instance, instead of applying an anatomical label to the subject data, the spatial origin of the subject data within a spatial subject atlas, may be defined. An example of this type of indication is illustrated in Fig. 3 via the lines that link the subject data 130_{1..7} to the corresponding positions 140_{1..7} in the graphical representation of the subject 210. In this case, the spatial origin of the subject data is used to retrieve the subject data. For instance, the subject atlas may be searched to find subject data in the vicinity of the access path.

In a related example, the subject data 130_{1..i} includes one or more sources of registered medical image data that are registered to a common subject coordinate system defining the origin of the source of subject data with respect to the subject atlas. In this example, the common subject coordinate system may be a 2D, or a 3D coordinate system. For instance, subject data in the form of both 2D, and also 3D, medical images data may be registered to a common volumetric coordinate system. For instance, subject data in the form of a 2D X-ray projection image of a subject may be registered to the 3D coordinate system, and also subject data in the form of a CT image of a subject may be registered to the 3D coordinate system. This has the effect of registering the 2D X-ray projection image to the CT image. The 3D coordinate system may be the coordinate system of the CT image. Registering the subject data to the common coordinate system facilitates a more straightforward retrieval of the subject data for different types of subject data along the access path because the spatial origins of the different types of subject data are inherently linked via the common coordinate system, and this makes it easier search the subject atlas to find subject data in the vicinity of the access path. It also facilitates the display of the subject data with reduced processing effort at the time of retrieving the subject data because the image data is inherently co-registered.

In another related example; the one or more sources of registered medical image data are registered to the access path 150, and the access path defines the origin of the source of subject data with respect to the subject atlas.

Registering the different sources of medical image data to the access path in accordance with this example facilitates a more straightforward retrieval of the subject data for different types of subject data along the access path because the spatial origins of the different types of subject data are inherently linked to the access path. By way of an example, CT image slices may be selected from a (surview) CT image and registered to their corresponding positions along an access path in a spatial coordinate system. Similarly, X-ray projection images may be registered to their corresponding positions along the access path in the spatial coordinate system. Together, the images provide a subject atlas wherein the images are registered to the access path. In this example, a CT image slice relating to a given position along the access path may be retrieved by performing a search for subject data relating to positions along the access path. The access path may be defined in the spatial coordinate system. For instance, the access path may be defined in a (surview) CT image, and the coordinate system of the (surview) CT image may serve as the spatial coordinate system.

In some examples, the subject data includes metadata, such as: a timestamp indicating a time and date at which the subject data was acquired, a field of view used to acquire a medical image, a viewing angle used to acquire a X-ray projection image, an image quality score for a medical image, an indication of whether or not a contrast agent was used to acquire a medical image, and so forth. The metadata may be used during the retrieving operation S110. For example, the timestamps may be used to select subject data for retrieval relating to a specified time interval. The viewing angles may be used to register X-ray projection image to other medical images.

The subject data that is retrieved in the operation S110 may be retrieved by the one or more processors 110 via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 2; in this operation, the subject data 130_{1..i}, is outputted. The subject data 130_{1..i} may be outputted in various ways, including to a display device, such as to the display 230 illustrated in Fig. 1, or to a virtual/ augmented reality display device, or to a printer, or to a computer-readable storage medium, or to the Internet, or to the Cloud, and so forth. In one example, the operation of outputting S120 the subject data 130_{1..i} includes outputting image data representing:
a graphical representation 210 of the subject including the access path 150;
a graphical representation of the subject data 130_{1..i}; and
an indication of the correspondence between the subject data 130_{1..i} and the one or more positions 140_{1..j} along the access path 150.

This example is described with reference to Fig. 3, which is an example of a graphical representation 210 of a subject and a graphical representation of subject data 130_{1..7} relating to one or more positions 140_{1..7} along an access path 150 to a treatment site 160 within the subject, in accordance with some aspects of the present disclosure. In the example illustrated in Fig. 3, the graphical representation 210 of the subject is provided in the form of a 2D outline of the subject. The graphical representation 210 of the subject includes the access path 150. In this example, the access path 150 extends from the entry point 190, which in this example is a point in the right femoral artery in the groin area, to the treatment site 160, which in this example is a stenosis, in the left anterior descending "LAD" artery. The access path 150 illustrated in Fig. 3 may be used to treat the stenosis in an interventional procedure such as balloon angioplasty combined with stenting, for example.

The example illustrated in Fig. 3 also includes a graphical representation of subject data 130_{1..7} relating to the positions 140_{1..7} along the access path 150. In the example illustrated in Fig. 3, the subject data includes a surview CT image 130₁ representing the torso of the subject, a surview MRI image 130₂ representing the torso of the subject, an MRI pelvic image 130₃ representing the pelvis of the subject, an IVUS image 130₄ representing a position in a coronary artery, a fluoroscopic X-ray projection image 130₅ representing a previously-implanted stent in a cardiac artery of the subject, a Calcium score 130₆ for a position in a coronary artery of the subject, and a CCTA image 130₇ representing a portion of the coronary arterial tree. The various types of subject data 130_{1..7} illustrated in Fig. 3 relate to the corresponding positions 130_{1..7} along the access path 150. The various types of subject data 130_{1..7} illustrated in Fig. 3 may be used to support the example balloon angioplasty procedure in the coronary artery. For instance, the surview CT image 130₁, the surview MRI image 130₂, and the MRI pelvic image 130₃, may be used to plan the access path 150. The IVUS image 130₄ may be used to diagnose the coronary artery for the presence of vascular disease. The fluoroscopic X-ray projection image 130₅ may be used to inform the physician of the location of the previously-implanted stent. The Calcium score for a position in a coronary artery 130₆ of the subject may be used to determine the type of interventional device, e.g. a type of atherectomy device, with which to treat the cardiac artery. The CCTA image 130₇ may be used to support the navigation of an interventional device to the treatment site, and also includes a measurement of the Instantaneous Wave-Free Ratio "IFR" and which provides a measure of the severity of coronary artery disease.

The example illustrated in Fig. 3 also includes an indication of the correspondence between the subject data 130_{1..i} and the one or more positions 140_{1..j} along the access path 150. This indication is provided in the example illustrated in Fig. 3 via the lines linking the various types of subject data 130_{1..7} to the positions 140_{1..7} along the access path 150.

The treatment site 160 illustrated in Fig. 3 is an example of an endoluminal treatment site. In general, a physician selects an entry point for reaching an endoluminal treatment site from a list of clinically-established options. The access path is then defined by the various anatomical lumens between the entry point and the treatment site. The anatomical lumen(s) may be identified in subject data that is acquired from a medical imaging system, e.g. via an image analysis technique such as image segmentation. In the example illustrated in Fig. 3, the treatment site 160 is an endovascular treatment site, and the access path 150 is consequently defined by one or more blood vessels within the vasculature between the entry point 190 and the treatment site 160. Other examples of entry points for reaching endovascular treatment sites in a coronary artery include a point in the left femoral artery in the groin area, or a point in the (left or right) radial artery. Other examples of entry points for reaching endovascular treatment sites in a coronary vein include a point in the internal jugular vein, or a point in the subclavian vein, or a point in the femoral vein, or a peripheral access point. For other endoluminal treatment sites, other entry points may be used. For instance, in order to reach a treatment site that is located in the respiratory tract, such as in the lung, a physician may use an entry point in the mouth. In order to reach a treatment site that is located in the gastrointestinal tract, the physician may use an entry point in the mouth, or the anus. Thereafter, the access path is defined respectively by the portion of the respiratory tract, or the portion of the gastrointestinal tract, between the entry point and the treatment site.

Further examples of the system 100 are described in the examples below.

In one example, the one or more processors 110 are configured to:
receive medical image data 170 representing an interventional device 180 within the subject; and
determine the one or more positions 140_{1..j} along the access path 150 based on a detected position of the interventional device 180 represented in the medical image data.

In this example, the medical image data may be acquired from various types of medical imaging systems. Thus, the medical image data may be CT image data, or X-ray projection image data, or MRI data, or PET image data, or SPECT image data, or ultrasound image data, or IVUS image data, or OCT image data, or (hyperspectral) endoscopic image data, for example.

In this example, in general, the interventional device may be any type of interventional device. In some examples, the interventional device may be an endoluminal device. For instance, the interventional device may be a catheter, or a guidewire, or a blood pressure sensing device, or a blood flow sensing device, or an IVUS imaging device, or an OCT imaging device, or an endoscopy device, or a colonoscopy device, or a bronchoscopy device, or a transesophageal echocardiogram "TEE" probe, and so forth.

In this example, the position of the interventional device 180 represented in the medical image data may be determined using various image processing techniques. For instance, a feature detector, or a neural network, may be trained to detect a position of a feature of the interventional device, such as a distal end of the interventional device, in the medical image data.

Having detected the position of the interventional device 180 represented in the medical image data, the detected position may be used to determine a position along the access path. This operation may be performed based on known transformations relating the received medical image data 170, and the subject data, to the access path 150. As described in the example above, these transformations may be known from the subject atlas. For instance, the received medical image data 170, and the subject data, may both be registered to a common coordinate system that defines an origin of the subject data with respect to a subject atlas. The access path may also be defined in the common coordinate system. Alternatively, the received medical image data 170, and the subject data, may both be registered to the access path that defines the origin of the source of subject data with respect to the subject atlas.

By way of an example, the received medical image data 170 may include a X-ray projection image that represents an interventional device in the form of a guidewire. and the subject data may include CT image slices from a (surview) CT image. The access path may be defined in the (surview) CT image. A CT image slice corresponding to the current position of the distal end of the guidewire along the access path may be retrieved from the database by the following operations: detect the position of the distal end of the guidewire in the X-ray projection image (e.g. using a feature detector, or a neural network), register the received X-ray projection image to the coordinate system of the (surview) CT image, determine the position of the distal end of the guidewire within the coordinate system of the (surview) CT image based on the detected position of the distal end of the guidewire in the X-ray projection image, and select the CT image slice corresponding to the current position of the distal end of the guidewire within the (surview) CT image. A graphical representation of the CT image slice corresponding to the current position of the distal end of the guidewire may then be outputted, e.g. to the display device 230 illustrated in Fig. 1. Alternatively, the subject data may include CT image slices from a (surview) CT image that are registered to corresponding positions along an access path that is defined in a coordinate system. In this case, a CT image slice corresponding to the current position of the distal end of the guidewire along the access path may be retrieved from the database by the following operations: detect the position of the distal end of the guidewire in the X-ray projection image (e.g. using a feature detector, or a neural network), register the received X-ray projection image to the access path, determine the position of the distal end of the guidewire along the access path based on the detected position of the distal end of the guidewire in the X-ray projection image, and select the CT image slice corresponding to the current position of the distal end of the guidewire along the access path. A graphical representation of the CT image slice corresponding to the current position of the distal end of the guidewire may then be outputted, e.g. to the display device 230 illustrated in Fig. 1.

Using the detected position of the interventional device represented in the medical image data to retrieve the subject data provides relevant subject data because the subject data relates to the position of the interventional device. Moreover, since the position of the interventional device is detected in medical image data, relevant subject data is outputted without the need to track the interventional device using a dedicated tracking system.

Other subject data may be retrieved, and outputted, in a similar manner. For instance, if the interventional device is located in particular coronary vessel branch, then for that branch, subject data such as the calcium score 130₆ illustrated in Fig. 3, or a previously-measured FFR value, geometric measurements of the vessel lumen, and so forth may be retrieved from the database 120 and outputted.

In one example, the positions of multiple interventional devices represented in the medical image data are determined and used to retrieve subject data corresponding to their respective positions.

In another example, the one or more processors 110 are configured to:
receive tracking data representing a tracked location of the interventional device 180 within the subject; and
determine the one or more positions 140_{1..j} along the access path 150 based on the received tracking data.

Thus, in this example, a tracking system is used to determine the position of the interventional device, and hence the positions along the access path for which the subject data is retrieved. Using the tracked location to retrieve the subject data provides relevant subject data because the subject data relates to the position of the interventional device. The tracking data may be provided by various types of tracking systems. For instance, an electromagnetic tracking system, or an optical fiber-based tracking system, may be used to track the location of the interventional device within a subject. An example of an electromagnetic tracking system that may be used, and which tracks a position of a magnetic marker, is disclosed in the document US2020/397510A1. An example of an optical fiber-based tracking system that may be used, and which uses a strain sensor to determine the position of an interventional device is disclosed in the document US2012/323115A1.

In this example, the tracking data represents the tracked location of the interventional device 180 within the subject in a tracking system coordinate system. The position(s) 140_{1..j} along the access path 150 may be determined based on the received tracking data by registering the coordinate system of the tracking system to the common coordinate system described above, e.g. to the common coordinate system that defines the origin of the subject data with respect to a subject atlas. This registration is typically known. For instance, it may be defined by the orientation of the subject with respect to the tracking system. It may also be known by performing a calibration operation wherein the interventional device is positioned at specified anatomical landmarks that are detectable in the subject atlas. This registration provides the tracked location of the interventional device in the common coordinate system, which may therefore be used to retrieve the subject data corresponding to the tracked location.

In one example, tracking data is received for multiple interventional devices within the subject, and the tracked locations of the devices are used to retrieve subject data corresponding to the respective positions of the devices.

In one example, the medical image data 170 comprises a live sequence of images representing the interventional device 180 within the subject. In this example, the operation of determining the positions 140_{1..j} along the access path 150 comprises repetitively detecting the position of the interventional device 180 in the images in the live sequence to provide a sequence of current positions along the access path 150. The outputting S120 comprises outputting the subject data 130_{1..i} relating to the current positions.

In this example, the subject data is updated in response to movements of the interventional device. Thus, it ensures that the outputted subject data remains relevant over time. In this example, the position of the interventional device 180 in the images in the live sequence is detected repetitively. The detection operation may be performed using the image processing techniques described above.

In one example, the tracking data comprises a live sequence of tracked locations of the interventional device 180 within the subject. In this example, the operation of determining the positions 140_{1..j} along the access path 150 comprises repetitively determining a current position of the interventional device 180 along the access path based on a current tracked location from the live sequence to provide a sequence of current positions along the access path. The outputting S120 comprises outputting the subject data 130_{1..i} relating to the current positions.

In this example, the subject data is likewise updated in response to movements of the interventional device. In this example, the current position of the interventional device 180 along the access path is determined repetitively. This operation may be performed using the tracking systems described above.

In a related example, the outputting operation S120 is also performed for a proximal position along the access path 150 and/or for a distal position along the access path 150. The proximal position is located a predetermined distance proximally along the path with respect to a current position. The distal position is located a predetermined distance distally along the path with respect to a current position.

This example provides the ability to look ahead, or behind, with respect to the current position of the interventional device, without the need to move the interventional device. It may be used to plan the navigation of an interventional device when approaching a bifurcation, or a bend in the vessel, for instance. In this example, the predetermined distance may be a predetermined distance along the access path, which may be curved, or it may be a linear distance. In the former case, the distance along the access path may be a distance along a centerline of the access path, for example. By way of some examples, the predetermined distance may be 2 centimeters, or 1 centimeter, or another distance.

In one example, the one or more processors 110 are configured to:
receive user input defining the one or more positions 140_{1..j} along the access path 150, and to retrieve the subject data 130_{1..i} relating to the one or more positions 140_{1..j} defined by the user input.

This example facilitates the retrieval of subject data relating to different positions 140_{1..j} along the access path without the need for an interventional device. This example may therefore be used to plan an interventional procedure. A user may for instance use the user input device to interact, e.g. via a cursor, with a displayed access path, and in response, the system 100 retrieves the subject data corresponding to the position defined by the user input device. In this example, the user input may be received via a user input device such as a mouse, a joystick, a touchscreen, and so forth. This example may be used instead of, or in addition to, the example described above in which the position(s) 140_{1..j} along the access path 150 are determined based on received medical image data, or based on received tracking data. In the latter case, it may be used to augment the subject data that is retrieved for the location of the interventional device.

In a related example, the received user input defines at least a current position along the access path 150. In this example, the outputting operation S120 is further performed for a proximal position along the access path and/or for a distal position along the access path, the proximal position being located a predetermined distance proximally along the path with respect to the current position, and the distal position being located a predetermined distance distally along the path with respect to the current position.

This example similarly provides the ability to look ahead, or behind, with respect to the current position of the interventional device without the need to move the interventional device.

In another example, the one or more processors 110 are configured to determine the access path 150 to the treatment site 160 within the subject.

In general, the access path may be determined based on medical image data representing the subject. The medical image data may be received by the one or more processors, e.g. from a medical imaging system, or it may be retrieved from the database 120. The received medical image data may be generated prior to, during, or in a peri-procedural phase of the interventional procedure. The medical image data may include a (surview) CT image, or a (surview) MRI image, representing the subject, for example. In some examples, the treatment site is in an endoluminal location within the subject, and at least a portion of the access path is defined by one or more anatomical lumens. The anatomical lumen(s) thus constrain the access path between the entry point and the treatment site. The lumen(s) may be identified in the medical image data using image segmentation techniques. For example, an endovascular access path may be determined from a (surview) CT image by: segmenting the image to determine the vasculature, identifying the treatment site, and one or more candidate entry points (e.g. using model-based segmentation techniques), and using a path-finding algorithm to determine one or more candidate access paths along the vasculature between the entry point(s) and the treatment site. The system may determine a single access path, or alternatively it may determine multiple candidate access paths. A user may then select one of the candidate access paths for use in the interventional procedure.

In one example, the one or more processors 110 are configured to:
receive user input identifying a type of the interventional procedure and/or the treatment site; and
determine the access path 150 based on the type of the interventional procedure and/or the treatment site 160, respectively.

In this example, user input is received that identifies a type of the interventional procedure and/or the treatment site. The user input may be received from a user input device such as described in the examples above. The user input may for instance be received via drop-down menu that lists a range of types of interventional procedures, e.g. PCI, balloon angioplasty, atherectomy; and/or the treatment site, e.g. coronary artery, coronary vein, and so forth. In this example, the user input is then used to determine the access path, based on the user input, using the technique described for the example above.

In one example, the subject data 130_{1..i} comprises one or more sources of registered medical image data. The source(s) of registered medical image data are registered to a common subject coordinate system; or the source(s) of registered medical image data are registered to the access path 150. In this example, the operation of determining an access path 150 to a treatment site 160 within a subject comprises:
retrieving, from the database 120, at least one of the one or more sources of registered medical image data for the subject; and
analyzing the at least one of the one or more sources of registered medical image data to determine the access path 150.

Determining the access path using registered medical image data facilitates the definition of the access path in a more straightforward manner. For instance, when multiple sources of subject data are analyzed in order to determine the access path, the registration facilitates a seamless transition in the access path between the different sources of subject data. In this example, the access path may be determined by analyzing the medical image data using the techniques described above.

In another example, the subject data 130_{1..i} comprises one or more sources of registered medical image data. The source(s) of registered medical image data are registered to a common subject coordinate system; or the source(s) of registered medical image data are registered to the access path 150. In this example, the operation of determining an access path 150 to a treatment site 160 within a subject comprises:
receiving medical image data 170 representing the subject;
registering the received medical image data 170 to the common subject coordinate system, or to the access path 150, respectively, to define the origin of the received medical image data 170 with respect to the subject atlas; and
analyzing the received medical image data 170 to determine the access path 150.

In this example, the registration of the received medical image data to the common subject coordinate system, or to the access path 150, facilitates a more straightforward retrieval of the subject data relating to the position(s) 140_{1..j} along the access path 150. In this example, the medical image data 170 may be received from a medical imaging system. The received medical image data may be generated prior to, during, or in a peri-procedural phase of the interventional procedure. In contrast to analyzing the subject data that is retrieved from the database, analyzing the received medical image data 170 enables the definition of the access path in a more recent representation of the subject's anatomy.

In one example, the one or more processors 110 are configured to:
receive medical image data 170 generated during the interventional procedure;
register the medical image data 170 to the common subject coordinate system, or to the access path 150, respectively, to define the origin of the received medical image data 170 with respect to the subject atlas; and
store the registered medical image data 170 in the database 120 to provide additional subject data for the subject of the interventional procedure.

Thus, this example provides additional subject data for the subject. This enables the system 100 to provide improved support to a physician in future interventional procedures. The medical image data 170 may be any of the types of medical image data described above. The registered medical image data 170 may be stored in a DICOM format, for example.

In a related example, if an interventional device is used during the procedure, the one or more processors 110 may detect the type of interventional device captured in the received medical image data 170 and/or a type of interventional procedure captured in the received medical image data 170; and store the type of interventional device and/or the type of interventional procedure captured in the received medical image data 170, in the database 120. The type of interventional device captured in the received medical image data 170 and/or a type of interventional procedure captured in the received medical image data 170, may be detected using image processing techniques. The type of interventional device captured in the received medical image data 170 and/or a type of interventional procedure captured in the received medical image data 170 may be stored in a DICOM format, e.g. as a report, for example.

In another example, the one or more processors 110 are configured to:
receive medical device data generated during the interventional procedure;
label the medical device data with an indication of an origin of the medical device data with respect to a subject atlas; and
store the labelled medical device data in the database 120 to provide additional subject data for the subject of the interventional procedure.

As in the previous example, this example provides additional subject data for the subject. This enables the system 100 to provide improved support to a physician in future interventional procedures. In this example, the labels may be applied using the techniques described above. Examples of medical device data in accordance with this example include blood pressure measurement data generated by a blood pressure sensing device, or blood flow measurement data generated by a blood flow sensing device, (hyperspectral) optical sensor data, and so forth.

In another example, the one or more processors 110 are configured to:
receive outcome data representing an outcome of the interventional procedure; and
store the outcome data in the database 120 to provide additional subject data for the subject of the interventional procedure.

In this example, storing the outcome data provides additional subject data for the subject. This enables the system 100 to provide improved support to a physician in future interventional procedures. In this example, the outcome data may include data indicating the success of the interventional procedure, side effects encountered following the interventional procedure, recommendations for future procedures, and so forth.

In another example, the one or more processors 110 are configured to:
generate model data representing an implantable device implanted in the subject or representing a treatment of the subject during the interventional procedure; and
store the model data in the database 120 to provide additional subject data for the subject of the interventional procedure.

In this example, storing the model data provides additional subject data for the subject. This enables the system 100 to provide improved support to a physician in future interventional procedures. An example of model data representing an implantable device is volumetric model data representing a stent that has been implanted in the subject. The model data may be incorporated in CT image data representing the subject's heart, for example. Examples of model data representing a treatment of the subject, include model data representing elements of a ballooning procedure, e.g. the inflation size of the balloon, or elements of a thrombectomy procedure, or elements of an embolization procedure.

In another example, the operation of outputting S120 the subject data 130_{1..i}, is performed selectively for the subject data 130_{1..i} relating to the one or more positions 140_{1..j}.

In this example, data from positions in the vicinity of the one or more positions 140_{1..j} is not outputted in the operation S120. Reducing the amount of subject data that is outputted in the operation S120 reduces the risk of over-burdening a physician with information.

In another example, the one more processors 110 are configured to degrade a visualization of, or to prune, the subject data 130_{1..i} outside of a region of interest surrounding the one or more positions 140_{1..j}.

This example similarly reduces the amount of subject data that is outputted in the operation S120, and likewise reduces the risk of over-burdening a physician with information.

In another example, the outputted image data comprises an indication of a current position of the interventional device 180.

Providing the current position of the interventional device in accordance with this example provides context to the outputted subject data. The current position of the interventional device may be determined from medical image data, or from tracking data, as described in the examples above. The current position of the interventional device may be provided in the graphical representation of the subject 210 illustrated in Fig. 3, for example.

In another example, the operation of outputting S120 the subject data 130_{1..i}, comprises outputting image data representing a historical interventional procedure performed at the treatment site 160 in the subject and/or an outcome of one or more historical interventional procedures performed in similar subjects.

The information that is outputted in accordance with this example helps to inform a physician, and may consequently improve the outcome of the current interventional procedure. For instance, outputting a historical image of an implanted stent may facilitate a comparison with a current state of the stent, and thereby inform a physician of the evolution of a stenosis that is treated by the stent over time. In this example, the image data representing a historical interventional procedure, and the outcome of one or more historical interventional procedures, may be retrieved from the subject data stored in the database.

In another example, for at least one of the one or more positions 140_{1..j}, the retrieving operation 5110 comprises retrieving, from the database 120, subject data 130_{1..i} relating to the position from a reference subject. In this example, the one or more processors 110 are configured to estimate the subject data 130_{1..i} relating to the position along the access path 150 within the subject, based on the retrieved subject data relating to the position from the reference subject.

In some situations, the subject data may be incomplete. For instance, previously-acquired medical image data may not have been acquired for all positions along the entire access path. For instance, IVUS image data may have been acquired for non-contiguous sections of the vasculature. This example addresses such situations by estimating the subject data in the gaps between the sections. The subject data is estimated from retrieved subject data relating to the position from the reference subject. The reference subject may for example be a subject having similar patient characteristics (e.g. gender, age, height, body mass index, and so forth). For instance, IVUS image data representing the missing sections may be used in place of the gaps in the subject data.

In another example, the system 100 also includes a computer readable storage medium storing the database 120. The database comprises the subject data 130_{1..i} relating to the subject of the interventional procedure.

The database 120 may be located locally, or remotely, with respect to the one or more processors 110. The database 120 may be distributed in the sense that it may be distributed across multiple computer readable storage media. In one example, the database comprises a Picture Archiving and Communication Systems "PACS" database. In one example, the database 120 is provided by the Cloud, as illustrated in Fig. 1.

It is noted that the system 100 may also include one or more of: a medical imaging system for providing the medical image data 170, such as for example the X-ray projection imaging system 220 illustrated in Fig. 1; a medical device, such as the guidewire 180 illustrated in Fig. 1; a display device, such as the display 230 illustrated in Fig. 1, for displaying the outputted subject data, other outputs generated by the one or more processors 110, and so forth; a patient bed 240; and a user input device (not illustrated in Fig. 1) configured to receive user input in relation to the operations performed by the one or more processors 110, such as a keyboard, a mouse, a touchscreen, and so forth.

In another example, a computer program product is provided. The computer program product comprises instructions which when executed by one or more processors, cause the one or more processors to carry out a method of supporting an interventional procedure to be performed at a treatment site within a subject, the method comprises:
retrieving S110, from a database 120, subject data 130_{1..i} for the subject of the interventional procedure, the subject data relating to one or more positions 140_{1..j} along an access path 150 to the treatment site 160 within the subject; and
outputting S120 the subject data 130_{1..i}.

In another example, a computer-implemented method of supporting an interventional procedure to be performed at a treatment site within a subject, is provided. The method comprises:
retrieving S110, from a database 120, subject data 130_{1..i} for the subject of the interventional procedure, the subject data relating to one or more positions 140_{1..j} along an access path 150 to the treatment site 160 within the subject; and
outputting S120 the subject data 130_{1..i}.

In another example, a medical imaging system 220 is provided. The medical imaging system comprises one or more processors 110 configured to:
retrieve S110, from a database 120, subject data 130_{1..i} for the subject of the interventional procedure, the subject data relating to one or more positions 140_{1..j} along an access path 150 to the treatment site 160 within the subject; and
output S120 the subject data 130_{1..j}.

In this example, the medical imaging system may be one of the example medical imaging systems described above.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, or by the medical imaging system, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for supporting an interventional procedure to be performed at a treatment site within a subject, the system comprising one or more processors (110) configured to:
retrieve (S110), from a database (120), subject data (130_{1..i}) for the subject of the interventional procedure, the subject data relating to one or more positions (140_{1..j}) along an access path (150) to the treatment site (160) within the subject; and
output (S120) the subject data (130_{1..i}).

2. The system according to claim 1, wherein the one or more processors (110) are further configured to:
receive medical image data (170) representing an interventional device (180) within the subject; and
determine the one or more positions (140_{1..j}) along the access path (150) based on a detected position of the interventional device (180) represented in the medical image data;
or
receive tracking data representing a tracked location of the interventional device (180) within the subject; and
determine the one or more positions (140_{1..j}) along the access path (150) based on the received tracking data.

3. The system according to claim 2, wherein the medical image data (170) comprises a live sequence of images representing the interventional device (180) within the subject, and wherein the determining the positions (140_{1..j}) along the access path (150) comprises repetitively detecting the position of the interventional device (180) in the images in the live sequence to provide a sequence of current positions along the access path (150), and wherein the outputting (S120) comprises outputting the subject data (130_{1..i}) relating to the current positions.

4. The system according to claim 2, wherein the tracking data comprises a live sequence of tracked locations of the interventional device (180) within the subject, and wherein the determining the positions (140_{1..j}) along the access path (150) comprises repetitively determining a current position of the interventional device (180) along the access path based on a current tracked location from the live sequence to provide a sequence of current positions along the access path, and wherein the outputting (S120) comprises outputting the subject data (130_{1..i}) relating to the current positions.

5. The system according to claim 1, wherein the one or more processors (110) are further configured to:
receive user input defining the one or more positions (140_{1..j}) along the access path (150); and wherein the one or more processors (110) are configured to retrieve the subject data (130_{1..i}) relating to the one or more positions (140_{1..j}) defined by the user input.

6. The system according to any previous claim, wherein the one or more processors (110) are further configured to determine the access path (150) to the treatment site (160) within the subject.

7. The system according to claim 6, wherein the one or more processors (110) are further configured to:
receive user input identifying a type of the interventional procedure and/or the treatment site; and
determine the access path (150) based on the type of the interventional procedure and/or the treatment site (160), respectively.

8. The system according to any previous claim, wherein the subject data (130_{1..i}) comprises an indication of an origin of the subject data with respect to a subject atlas; and
wherein the retrieving (S110) is performed based on the indication of the origin of the subject data.

9. The system according to claim 8, wherein the subject data (130_{1..i}) comprises one or more sources of registered medical image data, and wherein the one or more sources of registered medical image data are registered to a common subject coordinate system defining the origin of the source of subject data with respect to the subject atlas; or wherein the one or more sources of registered medical image data are registered to the access path (150) and wherein the access path defines the origin of the source of subject data with respect to the subject atlas.

10. The system according to claim 9 when dependent on claim 6, wherein the determining an access path (150) to a treatment site (160) within a subject comprises:
retrieving, from the database (120), at least one of the one or more sources of registered medical image data for the subject; and
analyzing the at least one of the one or more sources of registered medical image data to determine the access path (150);
or
receiving medical image data (170) representing the subject;
registering the received medical image data (170) to the common subject coordinate system, or to the access path (150), respectively, to define the origin of the received medical image data (170) with respect to the subject atlas; and
analyzing the received medical image data (170) to determine the access path (150).

11. The system according to claim 9, wherein the one or more processors (110) are further configured to:
receive medical image data (170) generated during the interventional procedure;
register the medical image data (170) to the common subject coordinate system, or to the access path (150), respectively, to define the origin of the received medical image data (170) with respect to the subject atlas; and
store the registered medical image data (170) in the database (120) to provide additional subject data for the subject of the interventional procedure.

12. The system according to any previous claim, wherein the outputting (S120) comprises outputting image data representing:
a graphical representation (210) of the subject including the access path (150);
a graphical representation of the subject data (130_{1..i}); and
an indication of the correspondence between the subject data (130_{1..i}) and the one or more positions (140_{1..j}) along the access path (150).

13. The system according to any previous claim, wherein for at least one of the one or more positions (140_{1..j}), the retrieving (S110) comprises retrieving, from the database (120), subject data (130_{1..i}) relating to the position from a reference subject; and
wherein the one or more processors (110) are configured to estimate the subject data (130_{1..i}) relating to the position along the access path (150) within the subject, based on the retrieved subject data relating to the position from the reference subject.

14. The system according to any previous claim, further comprising a computer readable storage medium storing the database (120), and wherein the database comprises the subject data (130_{1..i}) relating to the subject of the interventional procedure.

15. A computer program product comprising instructions which when executed by one or more processors, cause the one or more processors to carry out a method of supporting an interventional procedure to be performed at a treatment site within a subject, the method comprising:
retrieving (S110), from a database (120), subject data (130_{1..i}) for the subject of the interventional procedure, the subject data relating to one or more positions (140_{1..j}) along an access path (150) to the treatment site (160) within the subject; and
outputting (S120) the subject data (130_{1..i}).
